# EUROPEAN PATENT SPECIFICATION

(11) **EP 1 121 089 B1**
(45) Date of publication and mention of the grant of the patent: **08.03.2006**
(21) Application number: 99947470.3
(22) Date of filing: 27.09.1999
(51) Int. Cl.: A61K 8/89, A61Q 5/06

(54) **HAIR STYLING COMPOSITION**
HAARFORMUNGSZUSAMMENSETZUNG
FIXATEUR POUR CHEVEUX

(30) Priority: 14.10.1998 GB 9822419
(43) Date of publication of application: 08.08.2001
(73) Proprietor: UNILEVER PLC, London EC4P 4BQ (GB); UNILEVER N.V., 3013 AL Rotterdam (NL)
(72) Inventor: PRATLEY, Stuart Keith, Unilever Res.Port Sunlight, Wirral, Merseyside CH63 3JW (GB)
(74) Representative: Rots, Maria Johanna Francisca
(86) International application number: PCT/EP1999/007427
(87) International publication number: WO 2000/021493

(56) References cited:
- EP-A- 0 445 982
- EP-A- 0 818 190
- WO-A-96/31188
- WO-A-98/13011

## Description

### Field of the Invention

The present invention relates to hair styling compositions, for example creams, gels and especially aerosol hair styling mousse compositions, which contain cross-linked silicone and which deliver excellent styling as well as sensory feel.

### Background and Prior Art

Style creation products such as hair styling mousses provide human hair with a temporary set which can be removed by water or by shampooing, and function by applying a thin film of a resin or gum onto the hair to adhere adjacent hairs together so that they retain the particular shape or configuration at the time of application.

EP 818 190 describes how an emulsion polymerised silicone material having a particular, defined level of cross-linking, and which is cross-linked in emulsion form can be incorporated into a hair styling composition, such as a mousse, gel or cream, to give a formulation which delivers excellent style creation and longevity, whilst leaving the hair soft and natural. An important feature of these systems is the phase behaviour of the silicone, which is said to form a separate high viscosity aggregated phase in the composition. This phase behaviour is considered to be key to effective style creation.

A problem with "aggregating" systems as described in EP 818 190 is that they can tend to gel and form lumps under conditions of prolonged, high temperature storage.

Surprisingly, it has now been found that by the use of certain nonionic surfactants of specified HLB value, the silicone materials as described in EP 818 190 may be formulated into systems which do not form a separate high viscosity aggregated phase and yet nevertheless deliver effective style creation.

### SUMMARY OF THE INVENTION

The present invention provides a hair styling composition comprising:
(i) from 0.1% to 10% by weight, based on total weight, of a non-rigid emulsion polymerised cross-linked silicone polymer, in which the percentage of branched monomer units in the silicone polymer is from 0.05% to 10%;
(ii) from 0.1% to 10% by weight, based on total weight, of a hair styling polymer;
(iii) from 0.01% to 5% by weight, based on total weight, of a nonionic surfactant having an HLB value from 16 to 18
(iv) water; and
(v) from 0% to 30% by weight, based on total weight, of an aerosol propellant.

### DETAILED DESCRIPTION

### Cross-linked Silicone Polymer

The non-rigid emulsion-polymerised cross-linked silicone polymer (i) is present in compositions of the invention in an amount from 0.1% to 10% by weight based on the total weight of the composition, more preferably from 0.2% to 6% by weight, most preferably from 0.5 to 5% by weight.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross linking of the silicone polymer is typically introduced concurrently during emulsion polymerisation of the polymer through the inclusion of the required amount of trifunctional and tetrafunctional silane monomer units, for example, those of formula:
R Si (OH)₃ wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

Suitable emulsion polymerised cross-linked silicone polymers are commercially available or can be readily made using conventional techniques well known to those skilled in the art.

### Hair Styling Polymer

The hair styling polymer (ii) employed in compositions of the present invention should be capable of forming a film and holding the hair of the user in place.

Hair styling polymers are well known articles of commerce and many such polymers are available commercially which contain functional groups which render the polymers cationic, anionic, amphoteric or nonionic in character.

Examples of cationic hair styling polymers are copolymers of amino-functional acrylate monomers such as lower alkylaminoalkyl acrylate or methacrylate monomers such as dimethylaminoethyl methacrylate with compatible monomers such N-vinylpyrrolidone, vinyl caprolactam, or alkyl methacrylates such as methyl methacrylate and ethyl methacrylate and alkyl acrylates such as ethyl acrylate and n-butyl acrylate. Cationic hair styling polymers containing N-vinylpyrrolidone are commercially available from ISP Corporation such as those sold under the trademarks of Copolymer 845 and Copolymer 937 (copolymers of N-vinylpyrrolidone and t-butylaminoethyl methacrylate of average molecular weight about 1,000,000) and Gafquat® 734, 755 and 755N (quaternary ammonium polymers formed by the reaction of diethyl sulfate and a copolymer of N-vinylpyrrolidone and dimethylaminoethyl methacrylate and having the CTFA designation Polyquaternium-11).

Examples of anionic hair styling polymers are the copolymers of vinyl acetate and crotonic acid, terpolymers of vinyl acetate, crotonic acid and a vinyl ester of an alpha-branched saturated aliphatic monocarboxylic acid such as vinyl neodecanoate; copolymers of methyl vinyl ether and maleic anhydride (molar ratio about 1:1) wherein such copolymers are 50% esterified with a saturated alcohol containing from 1 to 4 carbon atoms such as ethanol or butanol; and acrylic copolymers, terpolymers, etc., containing acrylic acid or methacrylic acid as the anionic radical-containing moiety and esters of acrylic or methacrylic acid with one or more saturated alcohols having from 1 to 22 carbon atoms such as methyl methacrylate, ethyl acrylate, ethyl acrylate, ethyl methacrylate, n-butyl acrylate, t-butyl acrylate, n-butyl acrylate, t-butyl methacrylate, n-butyl methacrylate, n-hexyl acrylate, n-octyl acrylate, lauryl methacrylate and behenyl acrylate, glycols having from 1 to 6 carbon atoms such as hydroxypropyl methacrylate and hydroxyethyl acrylate, styrene, vinyl caprolactam, vinyl acetate, acrylamide, alkyl acrylamides and methacrylamides having 1 to 8 carbon atoms in the alkyl group such as methacrylamide, t-butyl acrylamide and n-octyl acrylamide, and other compatible unsaturated monomers. One specific example is the emulsion polymerised terpolymer of methacrylic acid, n-butyl acrylate and ethyl acrylate (e.g., in a weight percent ratio of 31:42:27, respectively). Another specific example is the Gantrez® ES series commercially available from ISP corporation (esterified copolymers of methyl vinyl ether and maleic anhydride).

Examples of amphoteric hair styling polymers are those which contain cationic groups derived from monomers such as t-butyl aminoethyl methacrylate as well as carboxyl groups derived from monomers such as acrylic acid or methacrylic acid. One specific example of an amphoteric hair styling polymer is Amphomer® sold by the National Starch and Chemical Corporation.

Examples of nonionic hair styling polymers are homopolymers of N-vinylpyrrolidone and copolymers of N-vinylpyrrolidone with compatible nonionic monomers such as vinyl acetate and terpolymers of ethyl acrylate, butyl methacrylate and methyl methacrylate. Nonionic polymers containing N-vinylpyrrolidone in various weight average molecular weights are available commercially from ISP Corporation such as homopolymers of N-vinylpyrrolidone having an average molecular weight of about 630,000 sold by ISP Corporation under the tradename PVP K-90 and those having an average molecular weight of about 1,000,000 sold under the tradename PVP K-120.

The hair styling polymers in compositions of the invention are most preferably selected from one or more ionic-type, i.e. cationic and/or anionic, hair styling polymers. Hair styling polymers selected from amphoteric and/or nonionic hair styling polymers may suitably be used in conjunction with these ionic-type hair styling polymers, to improve, for example, hair styling benefit.

Particularly preferred hair styling polymers in compositions of the invention are those ionic-type hair styling polymers selected from Polyquaternium-16, Polyquaternium 11, Polyquaternium 46 and esterified copolymers of methyl vinyl ether and maleic anhydride, optionally in combination with one or more nonionic hair styling polymers. Such nonionic hair styling polymers are preferably selected from vinylpyrrolidone homopolymers and especially copolymers of vinylpyrrolidone and vinyl acetate.

### Nonionic Surfactant

In addition to the cross-linked silicone polymer and the hair styling polymer, the hair styling composition of the invention also includes a nonionic surfactant (iii) in an amount ranging from 0.01% to 5%, preferably from 0.01% to 1%, most preferably from 0.02% to 0.8% by weight based on total weight.

The HLB (hydrophilic-lipophilic balance) is an important property of the nonionic surfactant (iii). The property per se and how it is calculated is described in J.Soc.cosmet.Chem.,1949,1,311. For a given nonionic surfactant, the HLB value represents the weight per cent of the hydrophilic content of the molecule divided by a factor of five.

Nonionic surfactants for use in compositions of the invention have an HLB (hydrophilic - lipophilic balance) value from 16 to 18.

Examples of suitable nonionic surfactants are condensation products of aliphatic (C₈-C₁₈) primary or secondary linear or branched chain alcohols or phenols with alkylene oxides, usually ethylene oxide and generally having at least 15, preferably at least 20, most preferably from 30 to 50 ethylene oxide groups. Other suitable nonionics include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Of particular use are those nonionic surfactants of general formula R(EO)x H, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

As some of the above-described nonionic surfactants form particularly stable foams, it may be preferable, depending on product form, to include anti-foam ingredients to reduce foaming to provide a foam stability preferred by the consumer. For example, in the case of hair styling mousse product forms, consumers generally prefer that the foam generated collapses after less than five minutes after discharge, for ease of spreading on the hair.

The invention accordingly provides, in a further aspect, a hair styling mousse incorporating an anti-foam ingredient.

By "anti-foam ingredient" is meant an agent which inhibits the build up of foam, or which reduces foam or entrapped air by causing the bubbles to burst, thus releasing the air. Most commercial anti-foam ingredients are mixtures of, inter alia, surface-active agents, hydrocarbons, alcohols, and polymers, to increase their effectiveness in multiple applications. Examples of anti-foaming agents suitable for personal care applications include bisphenylhexamethicone, dimethicone fluid, dimethiconol fluid, hexamethyldisiloxane, hexyl alcohol, isopropyl alcohol, petroleum distillates, phenethyl disiloxane, phenyl trimethicone, Polysilicone-7, propyl alcohol, silica dimethyl silylate, silica silylate, tetramethyl decynediol, trimethylsiloxysilicate, and mixtures thereof.

The selection of a suitable anti-foam ingredient will depend on the particular composition of the hair styling mousse concerned. In the case of hair styling mousses containing cross-linked silicones and nonionic surfactants as described above, it appears that silicone derived anti-foam ingredients are the most effective, especially those which do not contain too many hydrophilic groups, making them less effective, or too many hydrophobic groups making them poorly soluble. Typical levels would be 0.01% to 0.4%, ideally 0.04% to 0.2% although other levels may be suitable depending on type. Examples are the materials SL, SR, S575 and S369 from Wacker and MSA Compound from Dow Corning.

### Water

Compositions of the present invention will also include water, preferably distilled or deionised, as a solvent or carrier for the polymers and other components. Water will typically be present in amounts ranging from 30% to 98%, preferably from 60% to 95% by weight based on total weight.

Alcohol may optionally be employed as a co-solvent in compositions of the invention as this can enhance the performance of the styling composition. A suitable alcohol is an aliphatic straight or branched chain monohydric alcohol having 2 to about 4 carbon atoms. Isopropanol and especially ethanol are preferred. A suitable level for the alcohol is up to 20%, preferably from 5% to 15%, by weight based on total weight.

### Product Form

Compositions of the invention may suitably be in aerosol form. A particularly preferred product form is an aerosol hair mousse. Aerosol hair mousse compositions are emitted from the aerosol container as a foam which is then typically worked through the hair with fingers or a hair styling tool and either left on the hair or rinsed out.

Aerosol-form compositions of the invention will include an aerosol propellant (v) which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and isobutane. The propellants may be used singly or admixed. Water insoluble propellants, especially hydrocarbons, are preferred because they form emulsion droplets on agitation and create suitable mousse foam densities.

The amount of the propellant used is governed by normal factors well known in the aerosol art. For mousses the level of propellant is generally up to 30%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1% to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with polyallylsucrose as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of nonessential, optional components suitable for rendering the compositions more aesthetically acceptable or to aid use, including discharge from the container, of the product. Such conventional optional ingredients are well known to those skilled in the art, e.g. preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, and polymer plasticising agents such as glycerin and propylene glycol.

The invention also provides a method of styling hair by applying thereto a styling composition as is hereinabove described.

The following Examples further illustrate the preferred embodiments of the invention. All percentages referred to are by weight based on total weight unless otherwise indicated.

### Examples

The following Examples illustrate hair styling compositions according to the invention:

### Example 1

3% Polyquaternium 11 (added as HC Polymer 3A ex Osaka Organic)
3% Crosslinked silicone*
[* Emulsion polymerised dimethiconol containing 0.6% cross-linking, 55% aqueous emulsion, ex Dow Corning]
8% ethanol
0.3% Steareth-50 (added as NONION PS-2500, ex Nippon Oils & Fats)
8% LPG 3 (ex Taiyo Ekika Gas)
water, minors to 100%

### Example 2

1% Polyquaternium 11 (added as Luviquat PQ11 ex BASF)
3% Crosslinked silicone*
[* Emulsion polymerised dimethiconol containing 0.6% cross-linking, 55% aqueous emulsion, ex Dow Corning]
8% ethanol
0.3% Ceteareth-50 (added as Volpo CS-50, ex Croda Inc.) 0.05% MSA Cmpd (ex Dow Corning)
8% Cap 40 (ex Calor Gas)
water, minors to 100%

### Example 3

2% Polyquaternium 16 (added as Luviquat FC 550 ex BASF)
2% crosslinked silicone*
[* Emulsion polymerised dimethiconol containing 0.6% cross-linking, 55% aqueous emulsion, ex Dow Corning]
8% ethanol
0.3% Steareth-20 (added as Hetoxol STA-20 ex Heterene)
8% Cap 40 (ex Calor Gas)
water, minors to 100%

## Claims

1. A hair styling composition comprising:
(i) from 0.1% to 10% by weight, based on total weight, of a non-rigid emulsion polymerised cross-linked silicone polymer, in which the percentage of branched monomer units in the silicone polymer is from 0.05% to 10%;
(ii) from 0.1% to 10% by weight, based on total weight, of a hair styling polymer;
(iii) from 0.01% to 5% by weight, based on total weight, of a nonionic surfactant having an HLB value of from 16 to 18%
(iv) water; and
(v) from 0% to 30% by weight, based on total weight, of an aerosol propellant.

2. A hair styling composition according to claim 1, in which the cross-linked silicone polymer (i) is a cross-linked dimethiconol, having a percentage of branched monomer units in the silicone polymer in the range 0.15% to 7%.

3. A hair styling composition according to claim 1 or 2, in which the hair styling polymer (ii) is an ionic-type hair styling polymer selected from Polyquaternium 11, Polyquaternium 16, Polyquaternium 46, and salts of monoalkyl esters of poly(methyl vinyl ether/maleic acid) .

4. A hair styling composition according to any of claims 1 to 3 in which the nonionic surfactant (iii) has the general formula R(EO)x H, where R represents a straight or branched chain alkyl group having an average carbon chain length of 12-18 carbon atoms and x ranges from 30 to 50.

5. A hair styling composition according to any one of claims 1 to 4, which further comprises an alcohol selected from straight or branched chain monohydric alcohols having 2 to about 4 carbon atoms.

6. A hair styling composition according to any one of claims 1 to 5, which further comprises a silicone derived anti-foam ingredient.

7. A hair styling composition according to any of claims 1 to 6 which is an aerosol hair mousse in which the level of propellant (v) is from 2% to 30% by weight, based on total weight.

8. A hair styling composition according to claim 7 in which the propellant (v) is a hydrocarbon propellant selected from propane, n-butane, isobutane and mixtures thereof.

9. A hair styling composition according to any one of claims 1 to 7, which is a hair styling cream or gel including from 0.1% to 10% by weight based on total weight of a structurant or thickener.

## Patentansprüche

1. Haarstylingzusammensetzung, umfassend:
(i) 0,1 % bis 10 Gew.-%, bezogen auf das Gesamtgewicht, eines nicht starren, emulsionspolymerisierten, vernetzten Silikonpolymers, worin der Prozentsatz an verzweigten Monomereinheiten in dem Silikonpolymer 0,05 % bis 10 % ist;
(ii) 0,1 % bis 10 Gew.-%, bezogen auf das Gesamtgewicht, eines Haarstylingpolymers;
(iii) 0,01 % bis 5 Gew.-%, bezogen auf das Gesamtgewicht, eines nichtionischen Tensids mit einem HLB-Wert von 16 bis 18 %;
(iv) Wasser und
(v) 0 % bis 30 Gew.-%, bezogen auf das Gesamtgewicht, eines Aerosoltreibmittels.

2. Haarstylingzusammensetzung nach Anspruch 1, worin das vernetzte Silikonpolymer (i) ein vernetztes Dimethiconol mit einem Prozentsatz von verzweigten Monomereinheiten in dem Silikonpolymer in dem Bereich von 0,15 % bis 7 % ist.

3. Haarstylingzusammensetzung nach Anspruch 1 oder 2, worin das Haarstylingpolymer (ii) ein Haarstylingpolymer vom ionischen Typ, ausgewählt aus Polyquaternium 11, Polyquaternium 16, Polyquaternium 46 und Salzen von Monoalkylestern von Poly(methylvinylether/maleinsäure), ist.

4. Haarstylingzusammensetzung nach einem der Ansprüche 1 bis 3, worin das nichtionische Tensid (iii) die allgemeine Formel R(EO)xH aufweist, worin R eine gerad- oder verzweigtkettige Alkylgruppe mit einer mittleren Kohlenstoffkettenlänge von 12 bis 18 Kohlenstoffatomen wiedergibt und x im Bereich von 30 bis 50 liegt.

5. Haarstylingzusammensetzung nach einem der Ansprüche 1 bis 4, die weiterhin einen Alkohol, ausgewählt aus gerad- oder verzweigtkettigen einwertigen Alkoholen mit 2 bis etwa 4 Kohlenstoffatomen, umfasst.

6. Haarstylingzusammensetzung nach einem der Ansprüche 1 bis 5, die weiterhin einen von Silikon abgeleiteten Antischaumbestandteil umfassen.

7. Haarstylingzusammensetzung nach einem der Ansprüche 1 bis 6, die eine Aerosolhaarmousse darstellt, worin der Anteil an Treibmittel (v) 2 % bis 30 Gew.-%, bezogen auf das Gesamtgewicht, ist.

8. Haarstylingzusammensetzung nach Anspruch 7, worin das Treibmittel (v) ein Kohlenwasserstofftreibmittel, ausgewählt aus Propan, n-Butan, Isobutan und Gemischen davon, ist.

9. Haarstylingzusammensetzung nach einem der Ansprüche 1 bis 7, die eine Haarstylingcreme oder Gel ist, die/das 0,1 % bis 10 Gew.-%, bezogen auf das Gesamtgewicht, eines Strukturierungsmittels oder Verdickungsmittels einschließt.

## Revendications

1. Composition de fixateur pour cheveux comprenant :
(i) de 0,1 % à 10 % en poids, sur la base du poids total, d'un polymère de silicone réticulé polymérisé en émulsion non rigide, dans lequel le pourcentage de motifs monomères ramifiés dans le polymère de silicone est de 0,05 % à 10 % ;
(ii) de 0,1 % à 10 % en poids, sur la base du poids total, d'un polymère fixateur pour cheveux ;
(iii) de 0,01 % à 5 % en poids, sur la base du poids total, d'un tensioactif non ionique ayant une valeur du rapport HLB allant de 16 à 18 % ;
(iv) de l'eau ; et
(v) de 0 % à 30 % en poids, sur la base du poids total, d'un agent propulseur d'aérosol.

2. Composition de fixateur pour cheveux selon la revendication 1, dans lequel le polymère de silicone réticulé (i) est un diméthiconol réticulé, ayant un pourcentage de motifs monomères ramifiés dans le polymère de silicone dans la gamme de 0,15 % à 7 %.

3. Composition de fixateur pour cheveux selon la revendication 1 ou 2, dans lequel le polymère fixateur pour cheveux (ii) est un polymère fixateur pour cheveux de type ionique choisi parmi le polyquaternium 11, le polyquaternium 16, le polyquaternium 46, et des sels d'esters de monoalkyle de poly(éther de méthyle et de vinyle/acide maléique) .

4. Composition de fixateur pour cheveux selon l'une quelconque des revendications 1 à 3, dans lequel le tensioactif non ionique (iii) répond à la formule générale R(EO)_{×}H, dans laquelle R représente un groupe alkyle à chaîne linéaire ou ramifiée ayant une longueur de chaîne carbonée moyenne de 12 à 18 atomes de carbone et x est compris dans la gamme allant de 30 à 50.

5. Composition de fixateur pour cheveux selon l'une quelconque des revendications 1 à 4, lequel comprend en outre un alcool choisi parmi des alcools monohydriques à chaîne linéaire ou ramifiée ayant de 2 à environ 4 atomes de carbone.

6. Composition de fixateur pour cheveux selon l'une quelconque des revendications 1 à 5, lequel comprend en outre un ingrédient anti-mousse dérivé de la silicone.

7. Composition de fixateur pour cheveux selon l'une quelconque des revendications 1 à 6, lequel est une mousse d'aérosol fixateur pour cheveux dans laquelle le taux d'agent propulseur (v) est de 2 à 30 % en poids, sur la base du poids total.

8. Composition de fixateur pour cheveux selon la revendication 7, dans lequel l'agent propulseur (v) est un agent propulseur de type hydrocarbure choisi parmi le propane, le n-butane, l'isobutane et leurs mélanges.

9. Composition de fixateur pour cheveux selon l'une quelconque des revendications 1 à 7, lequel est une crème ou un gel fixateur pour cheveux comprenant de 0,1 % à 10 % en poids, sur la base du poids total, d'un agent structurant ou d'un agent épaississant.
